# EUROPEAN PATENT SPECIFICATION

(11) **EP 3 501 490 B1**
(45) Date of publication and mention of the grant of the patent: **30.08.2023**
(21) Application number: 17841550.1
(22) Date of filing: 17.08.2017
(51) Int. Cl.: A61K 8/60, A61K 31/7034, A61P 17/00, A61P 19/02, A61P 43/00, A61Q 19/08, A61Q 19/02

(54) **COMPOSITION FOR EXTERNAL USE THAT INCLUDES PTEROSTILBENE GLYCOSIDE**
PTEROSTILBENGLYCOSID-HALTIGE ZUSAMMENSETZUNG ZUR ÄUSSERLICHEN ANWENDUNG
COMPOSITION À USAGE EXTERNE CONTENANT UN PTÉROSTILBÈNE GLUCOSIDE

(30) Priority: 17.08.2016 JP 2016160127; 03.07.2017 JP 2017130528
(43) Date of publication of application: 26.06.2019
(73) Proprietor: Tomoike Bio Ltd., Shatin, N.T. (HK)
(72) Inventor: HAMADA, Hiroki, Okayama-shi Okayama 701-1205 (JP); UESUGI, Daisuke, Tamano-shi Okayama 706-0001 (JP); SHIMODA, Kei, Yufu-shi Oita 879-5503 (JP)
(74) Representative: Wynne-Jones IP Limited
(86) International application number: PCT/JP2017/029571
(87) International publication number: WO 2018/034328

(56) References cited:
- EP-A1- 2 832 344
- WO-A1-2018/021527
- JP-A- 2014 171 436
- JP-A- 2015 129 112
- US-A1- 2015 098 987
- US-A1- 2015 152 130
- US-A1- 2016 067 194
- DAISUKE SATO ET AL: "Synthesis of glycosides of resveratrol, pterostilbene, and piceatannol, and their anti-oxidant, anti-allergic, and neuroprotective activities", BIOSCIENCE, BIOTECHNOLOGY AND BIOCHEMISTRY, vol. 78, no. 7, 9 June 2014 (2014-06-09), pages 1123-1128, XP055470270, JP ISSN: 0916-8451, DOI: 10.1080/09168451.2014.921551
- Katie Schaefer: "Pterostilbene Skin Lightener and Antioxidant", , 31 August 2011 (2011-08-31), XP055668848, Retrieved from the Internet: URL:https://www.cosmeticsandtoiletries.com /formulating/function/active/128917933.htm l [retrieved on 2020-02-14]

## Description

### Technical Field

The present invention relates to the external use of a composition containing a pterostilbene glycoside.

### Background Art

Patent Literature (PTL) 1 discloses a composition containing carotenoid and pterostilbene as active ingredients. PTL 1 discloses that this composition is effective for preventing or reducing wrinkles, sagging, etc., of the skin.

Patent Literature (PTL) 2 discloses a composition containing protein particles and pterostilbene as active ingredients. PTL 2 discloses that this composition exerts a melanogenesis inhibition effect and can be used as a skin whitening agent.

Although the effect of preventing or reducing wrinkles, sagging, etc., of the skin achieved by the compound disclosed in PTL 1 is very attractive to the market, there is still room for improvement. The skin whitening effect of the composition disclosed in PTL 2 is also not sufficient to reach the level that is satisfactory for the market,

https://www.cosmeticsandtoiletries.com/formulating/function/ active/128917933.html discloses that pterostilbene is better absorbed than resveratrol, and has antioxidant, anti-inflammatory and skin lightening properties.

US2016067194 A1 discloses pterostilbene for treating, inhibiting or preventing uv-mediated loss of barrier function in skin, which includes the prevention/treatment of skin wrinkles and sagging etc.

### Citation List

### Patent Literature

PTL 1: JP2013-227275A
PTL 2: JP2013-075850A

### Summary of Invention

### Technical Problem

An object of the present invention is to provide a composition for external use that is effective for preventing or reducing wrinkles or sagging of the skin or a composition for external use that is effective for skin whitening or both of these compositions.

### Solution to Problem

The present inventors conducted extensive research and consequently found that a pterostilbene glycoside produced by subjecting pterostilbene to a specific treatment exhibits excellent effects in terms of preventing or reducing wrinkles, sagging, etc., of the skin. The present inventors also found that this glycoside also exhibits an excellent skin whitening effect.

### Advantageous Effects of Invention

The composition for external use of the present invention has a type IV collagen production-promoting effect. Based on this effect, the composition for external use of the present invention is useful for preventing sagging of the skin or imparting tightness to the skin or both.

The composition for external use of the present invention, in particular, pterostilbene monoglycoside, inhibits tyrosinase activity. Based on this effect, the composition for external use of the present invention is useful for exerting a skin whitening effect.

### Brief Description of Drawings

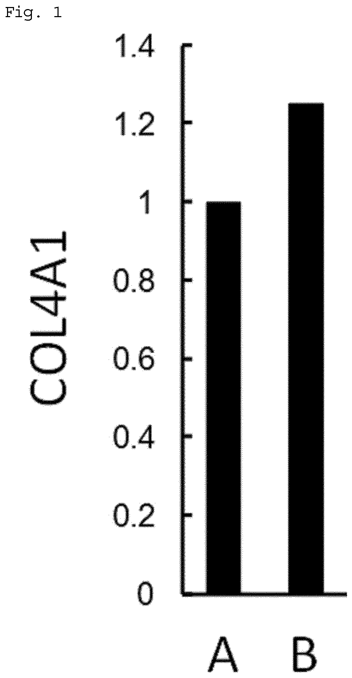

Fig. 1 is a graph showing the experimental results obtained using the microarray method for determining the amount of type IV collagen gene expression in skin cells (Experimental Example 1). A in the figure represents the results of a negative control experiment while B represents the results obtained with the addition of pterostilbene monoglucoside. The vertical axis of the graph in the figure denotes relative values of the amount of type IV collagen gene expression, relative to the amount of type IV collagen gene expression of A.

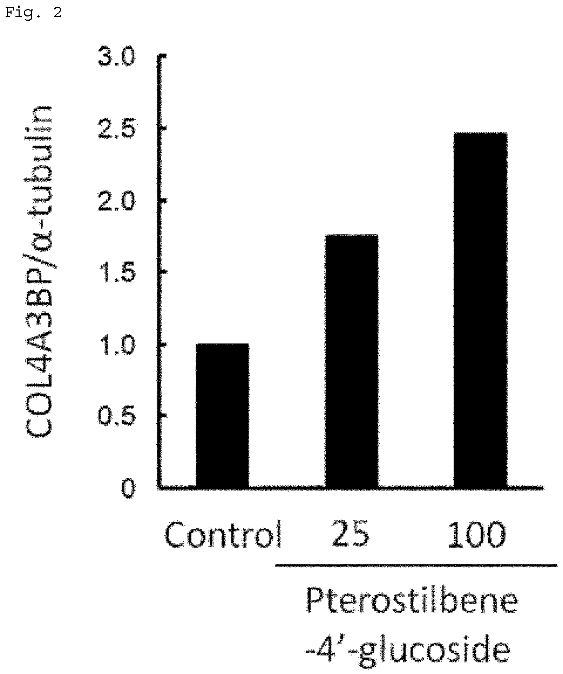

Fig. 2 is a graph showing the experimental results obtained by western blotting for determining the amount of type IV collagen expression in a three-dimensional skin model (Experimental Example 2). The vertical axis of the graph in the figure denotes values calculated by densitometry using α-tubulin as the internal standard for determining the amount of type IV collagen in the three-dimensional skin model. The horizontal axis of the graph in the figure is expressed in mM.

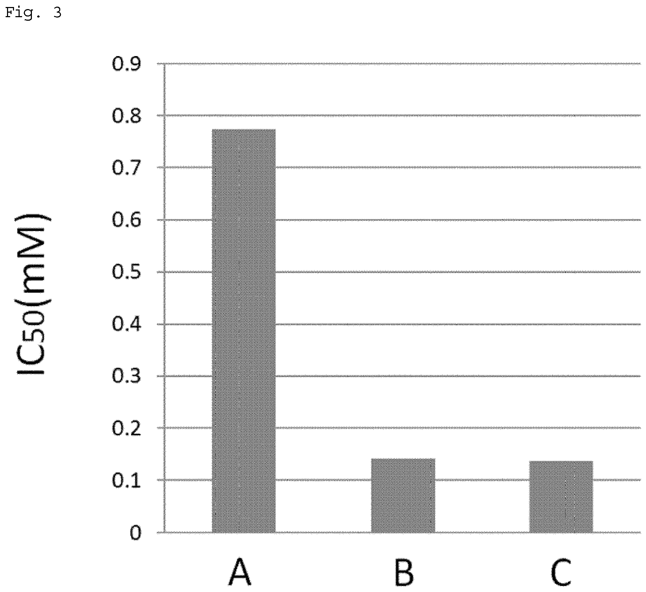

Fig. 3 is a graph showing the results of an *in vitro* tyrosinase inhibition experiment (Experimental Example 3). A in the figure represents the results obtained with the addition of pterostilbene, B represents the results obtained with the addition of pterostilbene monoglycoside, and C in the figure represents the results obtained with the addition of arbutin. The vertical axis of the graph in the figure denotes IC₅₀ values (mM) of tyrosinase.

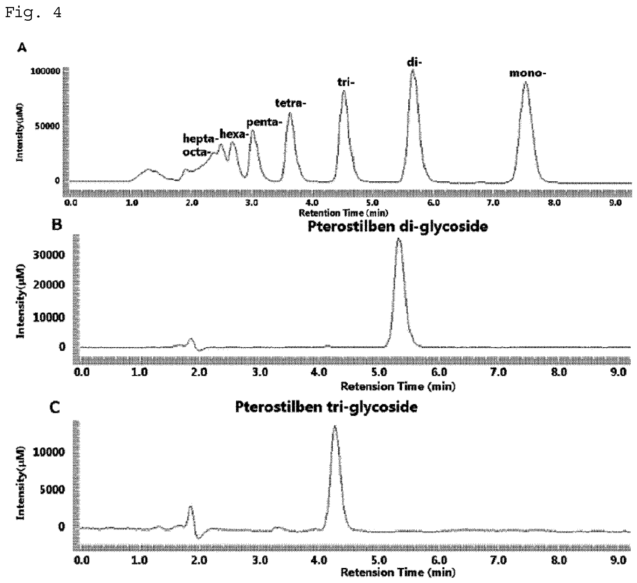

Fig. 4 shows the results of the production of pterostilbene polysaccharides as demonstrated in Experimental Example 4. A in the figure is a chart showing the results of HPLC analysis of a reactant obtained by reacting a pterostilbene monoglycoside with soluble starch in the presence of cyclodextrin glucanotransferase. The expression "mono-" in the figure represents "pterostilbene monoglycoside," "di-" represents "pterostilbene diglycoside," "tri-" represents "pterostilbene triglycoside," "tetra-" represents "pterostilbene tetraglycoside," "penta-" represents "pterostilbene pentaglycoside," "hexa-" represents "pterostilbene hexaglycoside," "hepta-" represents "pterostilbene heptaglycoside," and "octa-" represents "pterostilbene octaglycoside." B in the figure is a chart showing the isolation of pterostilbene diglycoside, based on the retention time. Likewise, C in the figure is a chart showing the isolation of pterostilbene triglycoside.

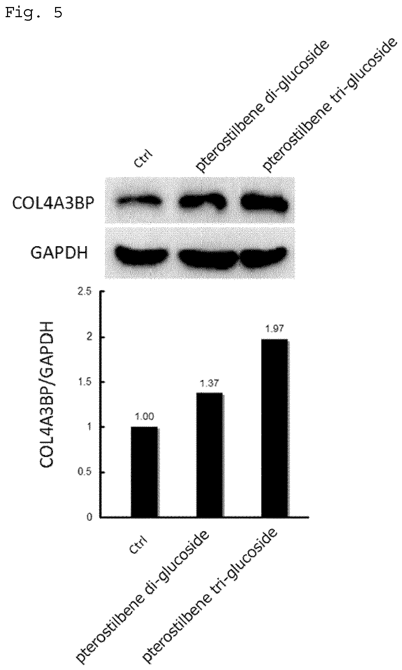

Fig. 5 is a graph showing the experimental results obtained by western blotting for determining the amount of type IV collagen expression in a three-dimensional skin model (Experimental Example 5). The photographic images in the figure show the results of western blotting. The vertical axis of the graph in the figure denotes the amount of type IV collagen expression determined by densitometry analysis using α-tubulin as the internal standard, based on the results of western blotting.

### Description of Embodiments

### Composition for External Use

The composition for the external use of the present invention contains a pterostilbene glycoside as an active ingredient.

The pterostilbene glycoside above is not particularly limited as long as the problems to be solved by the invention are solved. Examples include pterostilbene polysaccharides and pterostilbene monoglycosides.

### Pterostilbene Polysaccharide

The pterostilbene polysaccharide mentioned above is a glycoside obtained by modifying the hydroxyl group at position 3 of pterostilbene (IUPAC name: 4-[(E)-2-(3,5-dimethoxyphenyl)ethenyl]phenol) with a polysaccharide composed of two or more saccharides.

The saccharide residues contained in the pterostilbene polysaccharide are not particularly limited as long as the problems to be solved by the invention are solved. Examples of the saccharide residues include those from monosaccharides, such as glucose, threose, lyxose, allose, psicose, sedoheptulose, and volemitol. Among these saccharide residues, a glucose is preferred.

The saccharide residues may all be of the same type or different types as long as the problems to be solved by the invention are solved. For example, it is preferable that the saccharide residues are all of the same type.

The number of the saccharide residues present in the pterostilbene polysaccharide is not particularly limited as long as the problems to be solved by the invention are solved. For example, the number may be any of 2 to 10. The number is preferably 2 or 3, and most preferably 3.

The type of glycosidic linkage between the monosaccharides constituting the polysaccharide in the pterostilbene polysaccharide is not particularly limited as long as the problems to be solved by the invention are solved. Examples include 1-4 glycosidic linkage, 1-3 glycosidic linkage, 1-6 glycosidic linkage, and the like, with 1-4 glycosidic linkage being preferable. The linkage may also be α-glycosidic linkage or β-glycosidic linkage, with α-glycosidic linkage being preferable.

When multiple glycosidic linkages are present in the polysaccharide above, the glycosidic linkages may be of the same type or different types, and preferably of the same type.

The configuration of the monosaccharides constituting the saccharide residues present in the pterostilbene polysaccharide is not particularly limited as long as the problems to be solved by the invention are solved. Examples include D-configuration and L-configuration, with D-configuration being preferable.

Among these pterostilbene polysaccharides, compounds represented by the following formula (1): 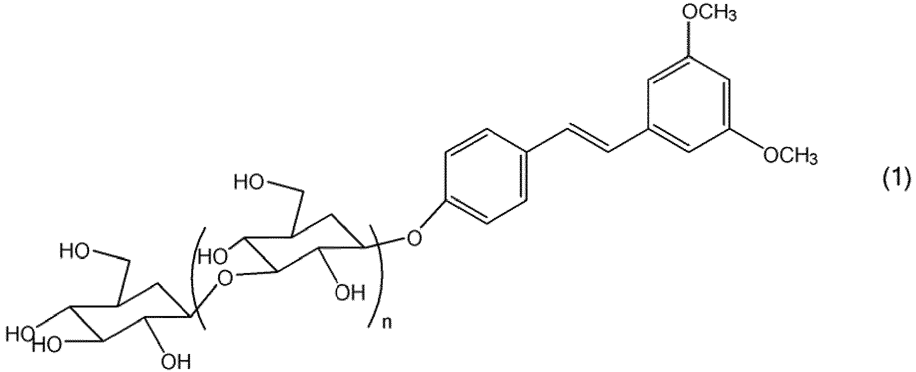 are most preferable.

In the formula, n is an integer of 1 to 9, preferably 1 to 5, and more preferably 1 to 2.

In the pterostilbene polysaccharide, the linkage pattern between the hydroxyl group at position 3 of pterostilbene and a saccharide is not particularly limited as long as the problems to be solved by the invention are solved. Examples include α-glycosidic linkage and β-glycosidic linkage, with β-glycosidic linkage being preferable.

The pterostilbene polysaccharide described above can be produced by a production method in which the pterostilbene monoglycoside mentioned later produced by chemical synthesis using the Koenigs-Knorr reaction, etc., using pterostilbene and a monosaccharide mentioned above as starting materials is used with one or more saccharides, along with an enzyme, such as cyclodextrin transferase; a method comprising subjecting both of the above starting materials to biosynthesis using a glycosyltransferase, such as PaGT2; or a method comprising subjecting a plant including fruits, such as grapes, to an extraction method that uses known processes.

### Pterostilbene Monoglycoside

The pterostilbene monoglycoside described above is a derivative obtained by modifying the hydroxyl group at position 3 of pterostilbene (IUPAC name: 4-[(E)-2-(3,5-dimethoxyphenyl)ethenyl]phenol) with a monosaccharide.

Monosaccharides, also called simple sugars, are saccharides that cannot be further hydrolyzed, and are not particularly limited as long as the problems to be solved by the invention are solved. Examples include glucose, threose, lyxose, allose, psicose, sedoheptulose, volemitol, and the like.

Among these monosaccharides, preferred is glucose. Therefore, the pterostilbene monoglycoside described above is preferably pterostilbene monoglucoside.

The configuration of the monosaccharide is not particularly limited as long as the problems to be solved by the invention are solved. Examples include D-configuration and L-configuration, with D-configuration being preferable.

In the pterostilbene monoglycoside, the linkage between the hydroxyl group at position 3 of pterostilbene and a monosaccharide is not particularly limited as long as the problems to be solved by the invention are solved. Examples include α-glycosidic linkage and β-glycosidic linkage, with β-glycosidic linkage being preferable.

Among these pterostilbene monoglycosides, most preferred is the pterostilbene monoglucoside represented by the following formula (2):

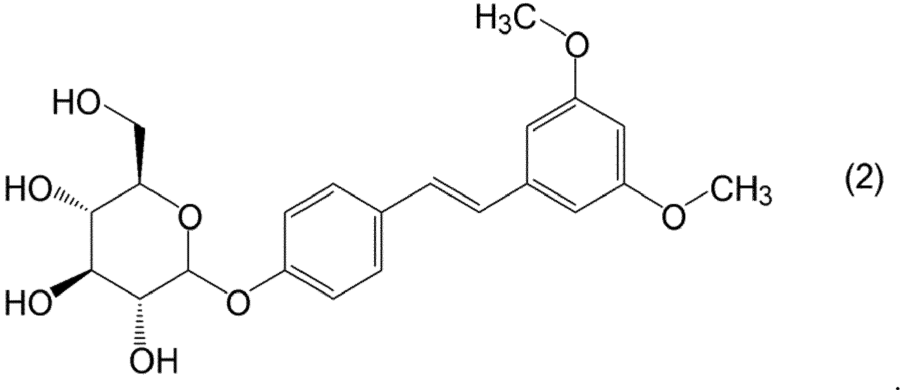

The composition for external use of the present invention can contain optional components, in addition to the pterostilbene polysaccharide. However, the composition for external use of the present invention preferably does not contain a carotenoid compound as an optional component.

Carotenoid compounds are derivatives of a compound having a chemical structure represented by the chemical formula: C₄₀H₅₆, and are compounds classified into carotenes, which consist only of carbon and hydrogen, and xanthophylls, which contain other elements in addition to carbon and hydrogen. Specific carotenes are not particularly limited as long as the problems to be solved by the invention are solved. Examples of the compounds include α-carotene, β-carotene, γ-carotene, δ-carotene, lycopene, and the like.

Xanthophylls are derivatives of a compound having a chemical structure represented by the chemical formula: C₄₀H₅₆, and are compounds containing carbon, hydrogen, and other elements. Specific xanthophylls are not particularly limited as long as the problems to be solved by the invention are solved. Examples include astaxanthin, zeaxanthin, lutein, cryptoxanthin, tunaxanthin, salmoxanthin, parasiloxanthin, violaxanthin, antheraxanthin, cucurbitaxanthin, diatoxanthin, alloxanthin, pectenol, pectenolone, mactraxanthin, capsanthin, capsanthinol, fucoxanthin, fucoxanthinol, peridinin, halocynthiaxanthin, amarouciaxanthin, canthaxanthin, echinenone, rhodoxanthin, bixin, norbixin, and the like. These xanthophylls also encompass monoesters and diesters thereof.

It is preferable that the composition for external use of the present invention essentially consists only of a pterostilbene glycoside as an active ingredient.

The active ingredient as used herein refers to a component that exerts a type IV collagen production-promoting effect or tyrosinase activity inhibitory effect or both, the effects of which are described later as effects achieved by the composition for external use of the present invention. That is, it is preferable that the composition for external use of the present invention does not contain a component that exerts a type IV collagen production-promoting action or a tyrosinase activity inhibitory action or both, other than the pterostilbene glycoside.

The amount of the pterostilbene glycoside contained in the composition for external use of the present invention is not particularly limited as long as the composition for external use of the present invention exhibits the effects of the present invention. For example, the amount of the active ingredient is usually about 0.001 to 100 mass%, per 100 mass% of the composition for external use.

As shown in the following Examples (e.g., Experimental Example 2), a composition that contains a pterostilbene glycoside as an active ingredient exhibits an excellent type IV collagen production-promoting effect. This suggests that the pterostilbene glycoside also exhibits an excellent type IV collagen production-promoting effect as well. Thus, a composition that contains the pterostilbene glycoside as an active ingredient is suitably used for promoting the production of type IV collagen.

Type IV collagen, also called a ceramide transfer protein (CETR), is found in the basal lamina of the skin and is known to be involved in the binding between extracellular matrices in the basal lamina. When a pterostilbene glycoside is applied externally, the production of type IV collagen is accelerated in the basal lamina while effects of reducing sagging of the skin caused by aging etc. and imparting tightness to the skin are exerted.

In view of the above, when applied (externally) to a human, the composition for external use of the present invention is expected to exert effects of reducing sagging of the skin and imparting tightness to the skin.

As shown in the following Examples (e.g., Experimental Example 3), pterostilbene monoglucoside exhibits an excellent tyrosinase inhibitory activity. Thus, other pterostilbene glycosides are expected to also exhibit an excellent tyrosinase inhibitory activity as well. Accordingly, a composition containing a pterostilbene glycoside as an active ingredient is suitably used for inhibiting tyrosinase activity.

Tyrosinase is an enzyme that is involved in melanin synthesis, and compositions that inhibit this activity are known to exert a skin whitening effect. Thus, when applied (externally) to a human, the composition for external use of the present invention is expected to exert a skin whitening effect. That is, the composition for external use of the present invention is useful as a whitening agent.

The subject to which the composition for external use of the present invention is applied is not limited as long as it is a human who is in need of the above effects.

In use, the amount of the composition for external use of the present invention is not particularly limited as long as the amount is within a range that can be expected to achieve the above effects. For example, the amount can be suitably determined based on the amount used as a cosmetic composition, a pharmaceutical composition, or the like, which are examples of a form of the composition for external use of the present invention as described later. Specifically, the amount may be usually about 1 to 5 mg per 1 cm² of skin or scalp, in terms of the amount of pterostilbene glycoside present in the composition for external use.

The form of the composition for external use of the present invention is not particularly limited as long as the problems to be solved by the invention are solved. Examples include cosmetic compositions and pharmaceutical compositions, with cosmetic compositions being preferable.

### Cosmetic Composition

The composition for external use of the present invention may be in the form of a cosmetic composition. The cosmetic composition of the present invention also contains quasi drugs, such as medicinal cosmetic products.

The amount of the pterostilbene glycoside present in the cosmetic composition is not particularly limited as long as the problems to be solved by the invention are solved. For example, the amount of the active ingredient is usually about 0.001 to 100 mass%, per 100 mass% of the cosmetic composition.

Specific embodiments of the cosmetic composition are not particularly limited as long as the problems to be solved by the invention are solved. Examples of the embodiments include make-up cosmetics, such as foundations, rouges, and face powders; basic cosmetics, such as face lotions, milky lotions, skin creams, lotions, oils, and packs; skin cleansing agents, such as facial washes, cleansing creams, and body soaps; hair cosmetics, such as shampoos, rinses, conditioners, styling agents, and hair restorers; bath additives, such as bath salts, bath tablets, and bath liquids; massage agents; cleaning agents; and the like.

The form of the cosmetic composition is not particularly limited as long as the problems to be solved by the invention are solved. Examples include lotions (liquids), mousses, gels, jellies, milky lotions, suspensions, creams, ointments, sheets, aerosols, sprays, and the like. With this types of forms, application is possible not only to the skin but also to scalp.

The amount of the cosmetic composition applied is not particularly limited as long as the problems to be solved by the invention are solved. Specifically, the amount can be suitably set on the basis of the gender and age of application subject, the application form of the cosmetic composition, the degree of desired effect, etc. For example, the amount may be usually about 1 to 5 mg per 1 cm² of the skin or scalp, in terms of the amount of the pterostilbene glycoside present in the cosmetic composition. The term "skin" refers to externally exposed regions in humans other than mucosae, except for scalp.

### Pharmaceutical Composition

The composition for external use of the present invention may be in the form of a pharmaceutical composition.

The amount of the pterostilbene glycoside present in the pharmaceutical composition is not particularly limited as long as the problems to be solved by the invention are solved. For example, the amount of the active ingredient is usually about 0.001 to 100 mass%, per 100 mass% of the pharmaceutical composition.

The dosage form of the pharmaceutical composition is not particularly limited as long as the problems to be solved by the invention are solved. Examples include those that are usually applied externally, such as aerosols, liquid agents, extracts, (ocular) ointments, percutaneous absorbents, suspensions, emulsions, spirits, patches, tincture, cataplasms, aromatic water agents, liniments, fluid extracts, and lotions.

The dosage of the pharmaceutical composition is not particularly limited as long as the problems to be solved by the invention are solved. Specifically, the dosage can be suitably set on the basis of the gender and age of application subject, the application form of the pharmaceutical composition, the degree of desired effect, etc. For example, the dosage may be usually about 1 to 5 mg/cm² for a human adult per day, in terms of the amount of pterostilbene glycoside contained in the pharmaceutical composition.

### Method for Producing Composition for External Use

The method for producing the composition for external use of the present invention comprises the step of mixing a pterostilbene glycoside and a component acceptable for compositions for external use.

The pterostilbene polysaccharide is as described above.

The component acceptable for compositions for external use is not particularly limited as long as the problems to be solved by the invention are solved. For example, the component may be a component acceptable for a cosmetic composition or a pharmaceutical composition, which are described above as examples of the forms of the composition for external use.

### Examples

Examples are provided below to describe the present invention in more detail. The present invention, however, is of course not limited to the following Examples.

### Experimental Example 1

The pterostilbene monoglucoside represented by the following formula (2): 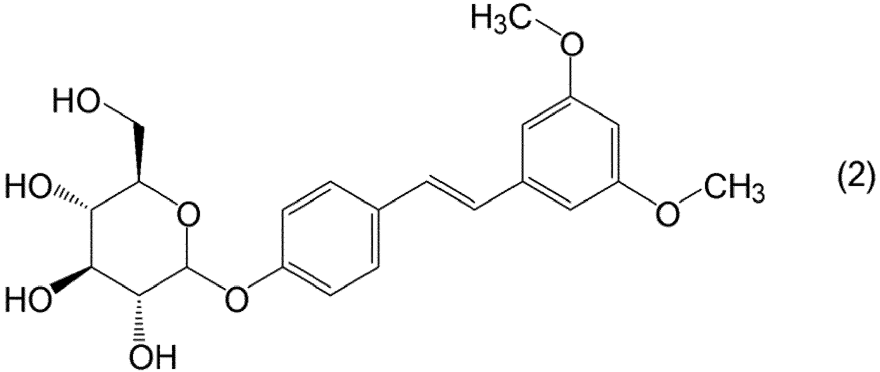 was obtained from Meisterbio Co., Ltd. and used in the following experiments.

Human skin cells were immortalized by introducing the hTERT gene and acclimated in a predetermined medium, Prigrow III (APB). Thereafter, the pterostilbene monoglucoside (50 µM) was added to the conditioned medium and cultured for 5 days in a 5% CO₂ environment at 37°C.

RNA was collected from the cells after culturing, and changes in the amount of type IV collagen mRNA expression were analyzed by microarray method. Fig. 1 shows the results. A negative control experiment was also performed in which the pterostilbene monoglucoside was not added to the conditioned medium.

Fig. 1 revealed that the addition of the pterostilbene monoglycoside (A in the figure) increased the expression amount of type IV collagen gene contained in the human skin cells by about 1.35 times, compared to the negative control experiment (B in the figure).

### Experimental Example 2

Since the results of Experimental Example 1 clarified that the pterostilbene monoglycoside increased the amount of type IV collagen gene expression in the skin cells, the pterostilbene monoglycoside was expected to also increase the amount of type IV collagen expression in the skin cells in the protein level as well.

To confirm this, an experiment was performed by western blotting using a kit (EFT-400; Kurabo Industries, Ltd.) for producing a three-dimensional skin model composed of human cutaneous keratinocytes and human fibroblasts.

The three-dimensional skin model produced following the protocol of the kit was acclimated in the medium supplied with the kit. The pterostilbene monoglucoside was added to the conditioned medium (25 µM and 100 µM at final concentrations) and cultured for 7 days.

The cells after culturing were collected, lysates thereof were subjected to western blotting using an anti-human collagen antibody (COL4A3BP; Abcam), and the amount of type IV collagen protein expression was analyzed. Further, western blotting using an anti-α-tubulin antibody as the internal standard was also performed, and the obtained image data was subjected to densitometry analysis. Fig. 2 shows the results.

The results of Fig. 2 clarified that the pterostilbene monoglucoside increased the amount of type IV collagen expression in the three-dimensional skin model by, at most, about 2.5 times in a concentration dependent manner, compared to the control. These results clarified that a composition containing the pterostilbene monoglucoside as an active ingredient is involved in various effects caused by an increase in type IV collagen in skin cells (e.g., an effect of imparting tightness to the skin by thickening of the basal lamina).

### Experimental Example 3

The tyrosinase inhibitory effect of pterostilbene monoglucoside was analyzed (IC₅₀ values were measured) based on a generally known method (e.g., Expert Opin Ther Pat. 2016; 26(3): 347-62). Tyrosinase (Cat. No. T3824, SIGMA) was used as tyrosinase. Further, pterostilbene (produced by Tokyo Chemical Industry Co., Ltd.; A in the figure), the pterostilbene monoglucoside above (B in the figure), and arbutin as a positive control (C in the figure) were used. Fig. 3 shows the results.

As is clear from Fig. 3, the pterostilbene monoglucoside (B in the figure) showed an excellent tyrosinase inhibitory activity, compared to pterostilbene (A in the figure), which is an aglycone thereof. When IC₅₀ values are used for comparison, the pterostilbene monoglucoside clearly achieved about a 5.5-times more excellent tyrosinase inhibitory effect than pterostilbene.

### Experimental Example 4

A pterostilbene polysaccharide was produced using the pterostilbene monoglucoside used in Experimental Examples 1 to 3 above as a starting material.

Specifically, 1 g of the pterostilbene monoglycoside represented by chemical formula (2) was dissolved in 40 mL of distilled water. To the thus-produced solution liquid, 4 g of soluble starch (Nacalai Tesque, Inc.) was added, followed by incubation at 60°C for 24 hours in the presence of 0.5 m cyclodextrin glucanotransferase (Amano Enzyme Inc.).

This enzyme catalyzes a reaction for binding a compound containing glucose molecules to other glucose molecules by 1-4 glycosidic linkage. The thus-obtained reactant was subjected to HPLC. Fig. 4A shows the results (HPLC charts). The following are HPLC conditions.

- Column used: YMC-Pack R&D ODS (ϕ 20 × 150 mm)
- Developing solution: 35% aqueous solution of acetonitrile
- Flow rate: 1 mL/min
- Measurement temperature: 40°C

As shown in Fig. 4A, the HPLC results confirmed that the reactant obtained above contained pterostilbene glycosides, i.e., pterostilbene monoglycoside and pterostilbene polysaccharides in which about 1 to 7 glucose units were α (1-4) linked to the pterostilbene monoglycoside. The results suggested a tendency that more glucose units were added to pterostilbene at a shorter retention time.

Further, pterostilbene diglycoside and pterostilbene triglycoside were isolated from this reactant by performing HPLC repeatedly. Fig. 4B (pterostilbene diglycoside) and Fig. 4C (pterostilbene triglycoside) show respective HPLC charts after isolation.

The following is mass spectrometry data of the isolated pterostilbene diglycoside and pterostilbene triglycoside. The mass analysis was performed using the ESI⁻ method.

Pterostilbene diglycoside:
   found: 579.2; estimate: 580.3
Pterostilbene triglycoside:
   found: 741.2; estimate: 742.3

### Experimental Example 5

An experiment was performed to confirm whether pterostilbene polysaccharides would increase the amount of type IV collagen protein expression in a three-dimensional skin model, using the pterostilbene diglycoside and pterostilbene triglycoside isolated in Experimental Example 4 above.

Specifically, an experiment was performed as in Experimental Example 2, and DMSO solutions were produced using the pterostilbene diglycoside or pterostilbene triglycoside mentioned above (both 50 µM), instead of using the pterostilbene monoglucoside. These solutions were added to three-dimensional skin models and cultured for 7 days. The lysates of cells after culturing were subjected to western blotting using an anti-human collagen antibody, and the amounts of type IV collagen expression in the cells were analyzed. As a negative control (Ctrl), an experiment was performed in which a DMSO solution was added alone to the three-dimensional skin model.

Further, western blotting using an anti-α-tubulin antibody as the internal standard was also performed, and the obtained image data was subjected to densitometry analysis. Fig. 5 shows the results.

The results shown in Fig. 5 revealed that the addition of pterostilbene diglycoside increased the amount of type IV collagen expression in the three-dimensional skin model by about 1.37 times, compared to the negative control (DMSO). The results also revealed that the addition of the pterostilbene diglycoside increased the amount of type IV collagen expression in the three-dimensional skin model by about 1.97 times.

These results clarified that a composition containing a pterostilbene polysaccharide as an active ingredient was also involved in various effects caused by an increase in type IV collagen in skin cells (e.g., an effect of imparting tightness to the skin by thickening of the basal lamina).

### Formulation Examples

Various cosmetic compositions were produced according to the formulation examples shown in Tables 1 to 4 below. The pterostilbene glycoside refers to a compound represented by the following formula (1): 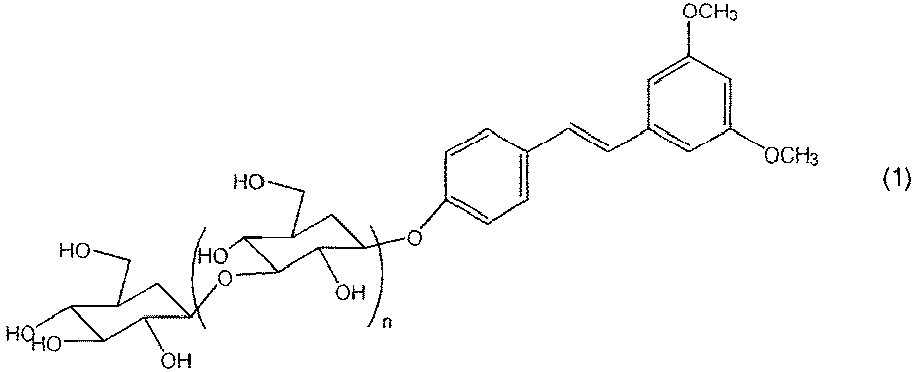 (wherein, n is 1 to 9, and trisaccharide is preferable; the glycosidic linkages between glucose units in the formula all represent 1-4 linkage; the linkage between position 3 of pterostilbene and glucose, and the glycosidic linkage between glucose units are both α-linkage); the compound represented by the following formula (2): 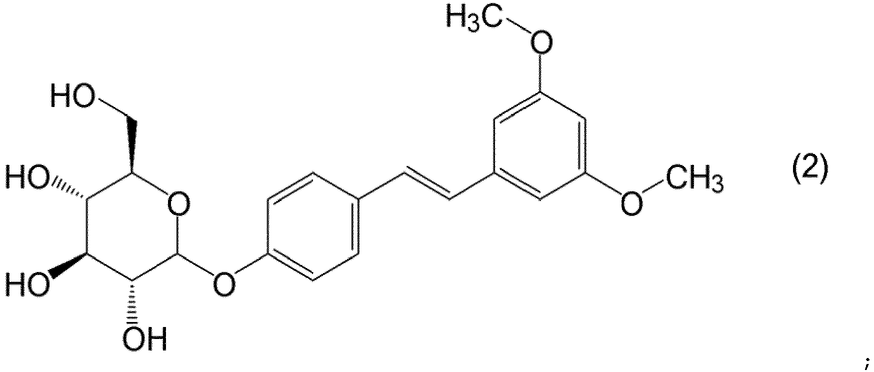 or both.

**Table 1**

| Formulation Example 1: Skin cream (40 g) | |
|---|---|
| Squalane | 15 wt% |
| Glycerol | 10 wt% |
| Betaine | 2 wt% |
| Cyclomethicone | 1.5 wt% |
| Trehalose | 1 wt% |
| BG | 4 wt% |
| Dimeticon | 1 wt% |
| 1,2-hexanediol | 0.5 wt% |
| Caprylyl glycol | 0.5 wt% |
| Pterostilbene glycoside | 0.5 wt% |
| Ubiquinone | 0.03 wt% |
| Orange flower extract | 0.1 wt% |
| Glucosamine hydrochloride | 0.2 wt% |
| PEG-20 sorbitan cocoate | 1 wt% |
| Dipotassium glycyrrhizinate | 0.05 wt% |
| Dextrin | 0.07 wt% |
| Carbomer | 0.9 wt% |
| Sodium carbonate | 0.45 wt% |
| Water | Balance |
| Total | 100 wt% |

**Table 2**

| Formulation Example 2: Lotion (120 mL) | |
|---|---|
| Glycerol | 10 wt% |
| Trehalose | 2 wt% |
| 1,2-hexanediol | 0.5 wt% |
| Caprylyl glycol | 0.5 wt% |
| BG | 5 wt% |
| Pterostilbene glycoside | 0.5 wt% |
| Hydrolyzed arginine | 0.5 wt% |
| Hydrolyzed collagen | 0.1 wt% |
| Orange flower extract | 0.1 wt% |
| Arginine ferulate | 0.1 wt% |
| Ubiquinone | 0.01 wt% |
| Dextrin | 0.02 wt% |
| PEG-20 sorbitan cocoate | 0.5 wt% |
| Dipotassium glycyrrhizinate | 0.05 wt% |
| Carbomer | 0.2 wt% |
| Sodium carbonate | 0.1 wt% |
| Water | Balance |
| Total | 100 wt% |

**Table 3**

| Formulation Example 3: Shampoo (300 mL) | |
|---|---|
| 25% Aqueous solution of sodium cocoyl glutamate | 25 wt% |
| 30% Aqueous solution of sodium methyl cocoyl taurate | 10 wt% |
| Lauryl betaine | 16 wt% |
| Cocamide DEA | 1 wt% |
| Betaine | 1 wt% |
| Polyquaternium-10 | 0.8 wt% |
| Pterostilbene glycoside | 0.5 wt% |
| EDTA-2Na | 0.1 wt% |
| Citric acid | 0.4 wt% |
| Methylparaben | 0.2 wt% |
| Purified water | Balance |
| Total | 100 wt% |

**Table 4**

| Formulation Example 4: Body soap (300 mL) | |
|---|---|
| 30% Aqueous solution of potash soap base | 50 wt% |
| 30% Aqueous solution of lauramidopropyl betaine | 15 wt% |
| 25% Aqueous solution of sodium laureth sulfate | 15 wt% |
| Glycol distearate | 1.5 wt% |
| 1,3-butylene glycol | 4 wt% |
| Pterostilbene glycoside | 0.5 wt% |
| Citric acid | q.s. wt% |
| Hydroxypropyl methylcellulose | 0.3 wt% |
| Purified water | Balance |
| Total | 100 wt% |

## Claims

1. External use of a composition to promote the production of type IV collagen, the composition containing a pterostilbene glycoside as an active ingredient.

2. External use of a composition to inhibit tyrosinase activity, the composition containing a pterostilbene glycoside as an active ingredient.

3. The external use according to claim 1 or 2, wherein the pterostilbene glycoside is a pterostilbene polysaccharide or a pterostilbene monoglycoside.

4. The external use according to claim 3, wherein the pterostilbene polysaccharide is represented by the following formula (1): 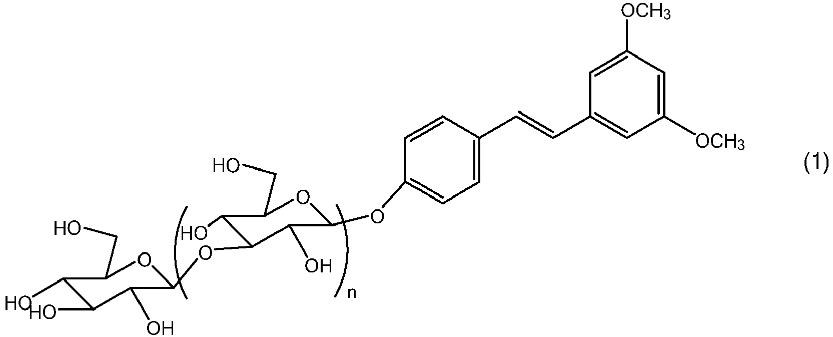 wherein n is an integer of 1 to 9.

5. The external use according to claim 4, wherein n is 1 or 2.

6. The external use according to claim 3, wherein the pterostilbene monoglycoside is pterostilbene monoglucoside.

7. The external use according to claim 6, wherein the pterostilbene monoglucoside is represented by the following formula (2):

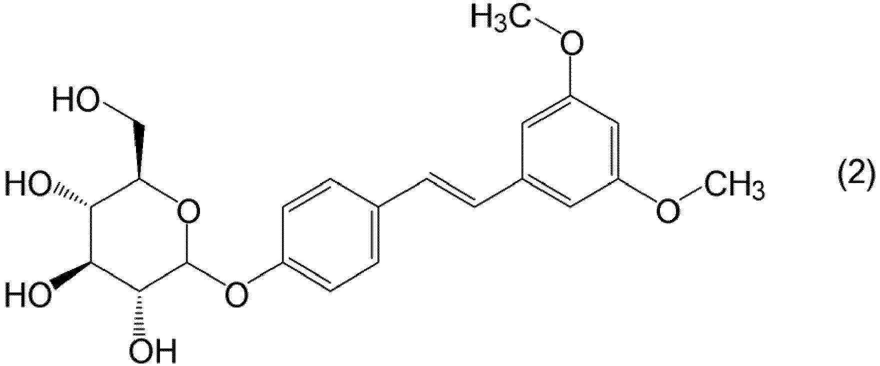

8. The external use according to any one of claims 1 to 7, which does not contain a carotenoid compound.

9. The external use according to any one of claims 1 to 8, wherein the active ingredient of the composition for external use essentially consists only of the pterostilbene glycoside.

10. The external use according to any one of claims 1 and 3 to 9, which is for preventing sagging of skin or imparting tightness to skin or both.

11. The external use according to any one of claims 2 to 10, which is for skin whitening.

12. The external use according to any one of claims 1 to 11, which is a cosmetic composition.

## Patentansprüche

1. Externe Verwendung einer Zusammensetzung, um die Produktion von Kollagen Typ IV zu fördern, wobei die Zusammensetzung ein Pterostilbene-Glycosid als einen Wirkstoff enthält.

2. Externe Verwendung einer Zusammensetzung, um Tyrosinaseaktivität zu hindern, wobei die Zusammensetzung ein Pterostilbene-Glycosid als einen Wirkstoff enthält.

3. Externe Verwendung nach Anspruch 1oder2 wobei das Pterostilbene-Glycosid ein Pterostilbene-Polysaccharid oder ein Pterostilbene-Monoglycosid ist.

4. Externe Verwendung nach Anspruch 3wobei das Pterostilbene-Polysaccharid durch die folgende Formel (1) dargestellt wird: 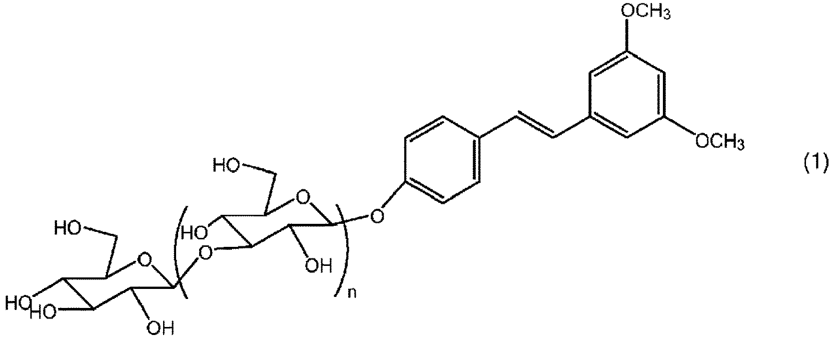 wobei n eine ganze Zahl von 1 bis 9 ist.

5. Externe Verwendung nach Anspruch 4, wobei n 1oder 2 ist.

6. Externe Verwendung nach Anspruch 3, wobei das Pterostilbene-Monoglycosid ein Pterostilbene-Monoglucosid ist.

7. Externe Verwendung nach Anspruch 6, wobei das Pterostilbene-Monoglucosid durch die folgende Formel (2) dargestellt wird:

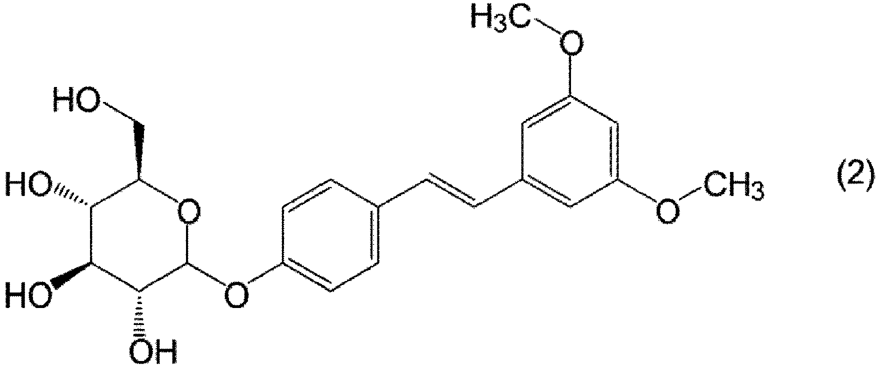

8. Externe Verwendung nach einem der Ansprüche 1 bis 7, wobei keine Carotinoid-Verbindung enthalten ist.

9. Externe Verwendung nach einem der Ansprüche 1 bis 8, wobei der Wirkstoff der Zusammensetzung für die externe Verwendung im Wesentlichen nur aus dem Pterostilbene-Glycosid besteht.

10. Externe Verwendung nach einem der Ansprüche 1 und 3 bis 9, was dazu dient, das Durchhängen von Haut zu verhindern oder der Haut Straffheit zu verleihen, oder beides.

11. Externe Verwendung nach einem der Ansprüche 2 bis 10, was für die Hautaufhellung dient.

12. Externe Verwendung nach einem der Ansprüche 1 bis 11, was eine kosmetische Zusammensetzung ist.

## Revendications

1. Utilisation externe d'une composition pour promouvoir la production de collagène de type IV, la composition contenant un glycoside de ptérostilbène en guise d'ingrédient actif.

2. Utilisation externe d'une composition pour inhiber l'activité de tyrosinase, la composition contenant un glycoside de ptérostilbène en guise d'ingrédient actif.

3. Utilisation externe selon la revendication 1 ou 2, dans laquelle le glycoside de ptérostilbène est un polysaccharide de ptérostilbène ou un monoglycoside de ptérostilbène.

4. Utilisation externe selon la revendication 3, dans laquelle le polysaccharide de ptérostilbène est représenté par la formule (1) suivante :, 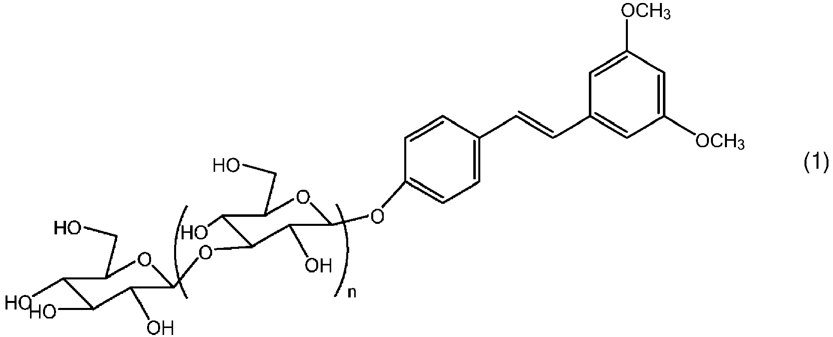 dans laquelle n est un nombre entier de 1 à 9.

5. Utilisation externe selon la revendication 4, dans laquelle n vaut 1 ou 2.

6. Utilisation externe selon la revendication 3, dans laquelle le monoglycoside de ptérostilbène est un monoglucoside de ptérostilbène.

7. Utilisation externe selon la revendication 6, dans laquelle le monoglucoside de ptérostilbène est représenté par la formule (2) suivante :

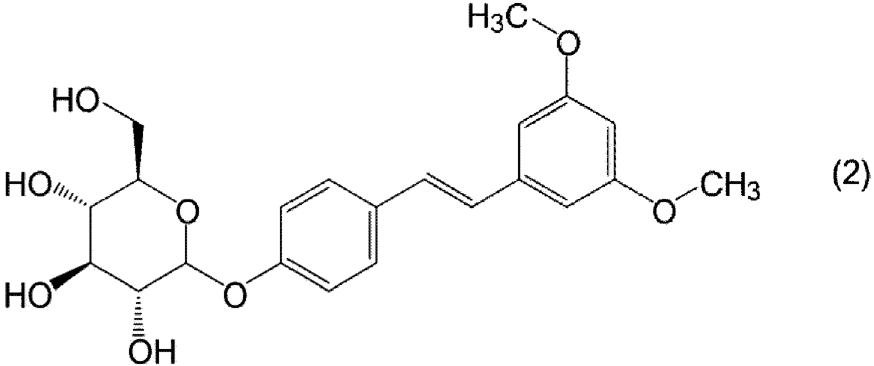

8. Utilisation externe selon l'une quelconque des revendications 1 à 7, qui ne contient pas de composé caroténoïde.

9. Utilisation externe selon l'une quelconque des revendications 1 à 8, dans laquelle l'ingrédient actif de la composition pour utilisation externe n'est constitué sensiblement que du glycoside de ptérostilbène.

10. Utilisation externe selon l'une quelconque des revendications 1 et 3 à 9, qui est destiné à prévenir un affaissement de la peau ou à donner de la fermeté à la peau ou l'un et l'autre.

11. Utilisation externe selon l'une quelconque des revendications 2 à 10, qui est destinée à un blanchiment de la peau.

12. Utilisation externe selon l'une quelconque des revendications 1 à 11, qui est une composition cosmétique.
